# EUROPEAN PATENT APPLICATION

(11) **EP 2 965 785 A1**
(43) Date of publication of application: **13.01.2016**
(21) Application number: 14176720.2
(22) Date of filing: 11.07.2014
(51) Int. Cl.: A61Q 19/00, A61Q 19/10, A61K 8/81

(54) **Novel use**

(71) Applicant: DSM IP Assets B.V., 6411 TE Heerlen (NL)
(72) Inventor: KLOCK, Jochen, 4303 Kaiseraugst (CH); SCHUELER, Goede, 4303 Kaiseraugst (CH); ROSENBERGER, Volker, 4303 Kaiseraugst (CH); RADSPIELER, Alexander, 4303 Kaiseraugst (CH)
(74) Representative: Berg, Katja

(57) **Abstract**

The present invention relates to skin cleansing composition comprising an anionic surfactant and an acrylic emulsion polymer obtainable by emulsion polymerization of a monomer composition consisting of a mixture of methacrylic acid, ethyl acrylate and n-butyl methacrylate, characterized in that the acrylic emulsion polymer is incorporated into the skin cleansing composition in a concentration ranging from 0.1 to 2 wt.-% based on the total weight of the composition. Furthermore the invention relates to the use of an acrylic emulsion polymer obtainable by emulsion polymerization of a monomer composition consisting of a mixture of methacrylic acid, ethyl acrylate and n-butyl methacrylate as skin conditioning agent.

## Description

The present invention relates to skin cleansing composition comprising an anionic surfactant and an acrylic emulsion polymer obtainable by emulsion polymerization of a monomer composition consisting of a mixture of methacrylic acid, ethyl acrylate and n-butyl methacrylate, characterized in that the acrylic emulsion polymer is incorporated into the skin cleansing composition in a concentration ranging from 0.1 to 2 wt.-% based on the total weight of the composition. Furthermore the invention relates to the use of an acrylic emulsion polymer obtainable by emulsion polymerization of a monomer composition consisting of a mixture of methacrylic acid, ethyl acrylate and n-butyl methacrylate as skin conditioning agent.

Shower gel formulations often contain substantial amounts of anionic surfactants to achieve effective cleansing, which, however, result in a sticky and unpleasant skin feel. Thus, there is an ongoing need for skin conditioning agents which are able to provide a soft and smooth skin feel when used in such shower gel formulations.

Surprisingly it has been found that an acrylic emulsion polymer obtainable by emulsion polymerization of a monomer composition consisting of a mixture of methacrylic acid, ethyl acrylate and n-butyl methacrylate is able to provide a smooth and soft skin feel when applied in a shower-gel formulation comprising an anionic surfactant.

Thus in a first embodiment the present invention relates to a skin cleansing composition comprising at least one anionic surfactant and an acrylic emulsion polymer obtainable by emulsion polymerization of a monomer composition consisting of a mixture of methacrylic acid, ethyl acrylate and n-butyl methacrylate, characterized in that the acrylic emulsion polymer is incorporated into the composition in a concentration ranging from 0.1 to 2 wt.-% based on the total weight of the composition.

In another embodiment the invention relate to the use of an acrylic emulsion polymer obtainable by emulsion polymerization of a monomer composition consisting of a mixture of methacrylic acid, ethyl acrylate and n-butyl methacrylate as skin conditioning agent.

The term skin conditioning agent as used herein is an agent which helps to keep the skin soft, smooth and pliable.

Preferably the amount of the acrylic emulsion polymer in the a skin cleansing composition is selected in the range of 0.25 to 1.8 wt.-%, preferably 0.4 to 1.5 wt.-%, most preferably in the range of 0.5 to 1.3 wt.-%, based on the total weight of the composition.

The acrylic emulsion polymers according to the invention are prepared by emulsion polymerization methods according to known methods as e.g. described in EP10193512.0 which is included herein by reference.

The method of free-radically initiated aqueous emulsion polymerization has been described previously on many occasions and is therefore sufficiently known to the person skilled in the art [cf. e.g. Encyclopedia of Polymer Science and Engineering, Vol. 8, pages 659 to 677, John Wiley & Sons, Inc., 1987; D. C. Blackley, Emulsion Polymerization, pages 155 to 465, Applied Science Publishers, Ltd., Essex, 1975; D. C. Blackley, Polymer Latices, 2.sup.nd Edition, Vol. 1, pages 33 to 415, Chapman & Hall, 1997; H. Warson, The Applications of Synthetic Resin Emulsions, pages 49 to 244, Ernest Benn, Ltd., London, 1972; D. Diederich, Chemie in unserer Zeit [Chemistry of our Time] 1990, 24, pages 135 to 142, Verlag Chemie, Weinheim; J. Piirma, Emulsion Polymerization, pages 1 to 287, Academic Press, 1982; F. Holscher, Dispersionen synthetischer Hochpolymerer [Dispersions of Synthetic High Polymers], pages 1 to 160, Springer-Verlag, Berlin, 1969 and DE-A 40 03422]. The free-radically initiated aqueous emulsion polymerization is usually carried out by dispersely distributing the monomers, usually with co-use of dispersants, in the aqueous medium, and polymerizing using at least one free-radical polymerization initiator.

Suitable free-radical polymerization initiators for the free-radical aqueous emulsion polymerization according to the invention are all those which are able to trigger a free-radical aqueous emulsion polymerization. These may in principle be either peroxides or azo compounds. Redox initiator systems are of course also suitable. Peroxides which may be used are, in principle, inorganic peroxides, such as hydrogen peroxide or peroxodisulfates, such as the mono- or di-alkali metal or ammonium salts of peroxide disulfuric acid, for example, its mono- and di-sodium, -potassium or ammonium salts or organic peroxides, such as alkyl hydroperoxides, for example tert-butyl, p-menthyl or cumyl hydroperoxide, tert-butyl perpivalate, and dialkyl or diaryl peroxides, such as di-tert-butyl or di-cumyl peroxide, 2,5-dimethyl-2,5-di(t)butyl-peroxy(hexane) or dibenzoyl peroxide.

The azo compounds used are essentially 2,2'-azobis(isobutyronitrile), 2,2'-azobis(2,4-dimethylvaleronitrile) and 2,2'-azobis(amidinopropyl) dihydrochloride (AIBA, corresponds to V-50.TM. from Wako Chemicals), 1,1'-azobis(1-cyclohexanecarbonitrile), 2,2'-azobis(2-amidinopropane)salts, 4,4'-azobis(4-cyanovaleric acid) or 2-(carbamoylazo)isobutyronitrile.

Suitable oxidizing agents for redox initiator systems are essentially the abovementioned peroxides. Corresponding reducing agents which may be used are sulfur compounds with a low oxidation state, such as alkali metal sulfites, for example potassium and/or sodium sulfite, alkali metal hydrogensulfites, for example potassium and/or sodium hydrogen sulfite, alkali metal metabisulfites, for example potassium and/or sodium metabisulfite, formaldehyde sulfoxylates, for example potassium and/or sodium formaldehyde sulfoxylate, alkali metal salts, specifically potassium and/or sodium salts, of aliphatic sulfinic acids (i.e. Bruggolite^{®} FF6) and alkali metal hydrogen sulfides, such as, for example, potassium and/or sodium hydrogen sulfide, salts of polyvalent metals, such as iron(II) sulfate, iron(II) ammonium sulfate, iron(II) phosphate, enediols, such as dihydroxymaleic acid, benzoin and/or (i-) ascorbic acid, and reducing saccharides, such as sorbose, glucose, fructose and/or dihydroxyacetone.

The initiators are usually used in amounts up to 10% by weight, preferably 0.02 to 5% by weight, based on the monomers to be polymerized.

Surfactants can be utilized in order to assist the dispersion of the polymer in water. Suitable surfactants include but are not limited to conventional anionic and/or non-ionic surfactants and mixtures thereof such as Na, K and NH₄ salts of dialkylsulphosuccinates, Na, K and NH₄ salts of sulphated oils, Na, K and NH₄ salts of alkyl sulphonic acids, Na, K and NH₄ alkyl sulphates, alkali metal salts of sulphonic acids; fatty alcohols, ethoxylated fatty acids and/or fatty amides, and Na, K and NH₄ salts of fatty acids such as Na stearate and Na oleate. Other anionic surfactants include alkyl or (alk)aryl groups linked to sulphonic acid groups, sulphuric acid half ester groups (linked in turn to polyglycol ether groups), phosphonic acid groups, phosphoric acid analogues and phosphates or carboxylic acid groups. Non-ionic surfactants include polyglycol ether compounds and preferably polyethylene oxide compounds as disclosed in "Non-Ionic Surfactants - Physical Chemistry" edited by M.J. Schick, M. Decker 1987. The amount of surfactant used is preferably 0 to 15 wt.-% by, more preferably 0 to 8 wt-%, still more preferably 0 to 5% wt.-%, especially 0.1 to 3 wt-% and most especially 0.3 to 2 wt-% on the total weight of vinyl monomers required.

Chain transfer agent may be added to control the molecular weight. Suitable chain transfer agents include mercaptans such as n-dodecylmercaptan, n-octylmercaptan, t-dodecylmercaptan, mercaptoethanol, iso-octyl thioglycolate, C₂ to C₈ mercapto carboxylic acids and esters thereof such as 3-mercaptopropionic acid and 2-mercaptopropionic acid. Mixtures of two or more regulators may also be used.

Preferably 0.05 to 5 wt-%, more preferably 0.1 to 3 wt-% and most preferably 0.1 to 1 wt-% of chain transfer agent based on the weight of vinyl monomers required is used. The alkanethiols are usually added to the polymerization together with the monomers.

If, in the polymerization, thiols are used, a subsequent hydrogen peroxide treatment could be required in order to obtain polymers with a neutral odor.

The emulsion polymerization usually takes place with the exclusion of oxygen, for example under a nitrogen or argon atmosphere, at temperatures in the range from 20 to 200°C. Polymerization temperatures in the range from 50 to 130°C, in particular 70 to 95°C are advantageous.

The polymerization can be carried out batch-wise, semi-continuously or continuously. The polymerization and the monomer and regulator feed are often carried out semi-continuously by the feed method. Preferably, at least some of the monomers, initiators and, if appropriate, regulators are metered into the reaction vessel uniformly throughout the polymerization. However, it is also possible to have an initial charge of the monomers and the initiator in the reactor and to polymerize them, with cooling if appropriate. Another option is to carry out the polymerization using seed latex prepared from the polymers to be polymerized in the first polymerization phase. The remainder of the monomer mixture is added, preferably by the feed method.

The polymerization reaction advantageously takes place until the monomer conversion is >95% by weight, preferably >98% by weight or >99% by weight.

It is often useful if the aqueous polymer dispersion obtained is subjected to an after-polymerization step in order to reduce further the amount of unreacted monomer. This measure is known to the person skilled in the art (for example EP-B 3957, EP-B 28348, EP-B 563726, EP-A 764699, EP-A 767180, DE-A 3718520, DE-A 3834734, DE-A4232194, DE-A 19529599, DE-A 19741187, DE-A 19839199, DE-A 19840586, WO 95/33775 or U.S. Pat. No. 4,529,753). It is of course also possible to subject the aqueous polymer dispersion obtained to an inert-gas and/or steam stripping, likewise known to the person skilled in the art, before or after the after-polymerization step. This stripping operation preferably takes place after the after-polymerization step. As is described in EP-A 805169, partial neutralization of the dispersion to a pH in the range from 5 to 7, preferably to a pH in the range from 5.5 to 6.5, is advantageous before the physical deodorization.

If applicable, due to a low monomer content after preparation, these possible additional steps can be omitted and the dispersions can be further used as such providing an economical advantage.

In a preferred embodiment, the aqueous polymer dispersion obtained is subjected to a post initation (post treatment/ after-polymerization) using t-butyl hydroperoxide with iso-ascorbic acid or t-butyl hydroperoxide with aliphatic sulfinic acids (i.e. Bruggolite^{®} FF6) in water. Particular preferred is a post-treatment with t-butyl hydroperoxide with aliphatic sulfinic acids (i.e. Bruggolite^{®} FF6) as this reduces possible discoloration of the emulsion polymer as such or when dissolved in the end formulation.

Particularly preferred acrylic emulsion polymers according to the present invention are the ones obtainable by emulsion polymerisation of a monomer composition consisting essentially of 10-30 wt.-% of methacrylic acid, 5-15 wt.-% of ethyl acrylate and 60-80 wt.-% of n-butyl methacrylate, such as in particular of 15-25 wt.-% of methacrylic acid, 8-12 wt.-% of ethyl acrylate and 65-75 wt.-% of n-butyl methacrylate, such as even more in particular of 18-23 wt.-% of methacrylic acid, 9-11 wt.-% of ethyl acrylate and 67-72 wt.-% of n-butyl methacrylate.

The term 'a monomer composition consisting essentially of' as used according to the present invention means that the total amount of monomer ideally sum up to 100 wt.-%. It is however not excluded that small amount of impurities or additives may be present such as e.g. in amounts of less than 5 wt.-%, preferably less than 3 wt.-% which are e.g. introduced via the respective raw materials.

Furthermore, the acrylic emulsion polymers according to the present invention preferably have a molecular weight between 30-500 kDalton, more preferably 50 - 250 kDalton and most preferred between 75 and 200 kDalton such as in the range of 100 to 150 kDalton, and a glass transition temperature (Tg) between 40 and 140°C, more preferably between 55 and 130 °C and most preferred between 70-120 °C such as e.g. between 70 and 100 °C.

The Acid value is preferably selected in the range of 100-200 mgKOH/g, more preferably 120-180 mg KOH/g and most preferably in the range of 140-160 mg KOH/g.

Advantageously the acrylic emulsion polymers according to the invention have a molecular weight between 75 -200 kDalton, an acid value in the range of 120-180 mg KOH/g and a Tg in the range of 70-120 °C such as in particular a molecular weight between 100-150 kDalton, an acid value in the range of 120-180 mg KOH/g and a Tg in the range of 70-100 °C.

The acrylic emulsion polymer can be used in isolated form (as solids) or jn the form of an aqueous dispersion which is e.g. obtainable from the emulsion polymerisation.

Preferably an aqueous dispersion of the acrylic emulsion polymer is used, more preferably the aqueous dispersion obtained from emulsion polymerization (eventually comprising a post treatment step) is used directly in the preparation of the skin cleansing composition according to the invention. It is well understood to a person skilled in the art that the amount of the aqueous dispersion to be added to the skin cleansing composition according to the present invention has to be adjusted based on the solid content (i.e. content of the acrylic emulsion polymer).

Preferably the aqueous dispersion used for the preparation of the skin cleansing composition according to the present invention has a solid content selected in the range of 30 to 50 wt.-% such as in the range of 35 to 45 wt.-% and a pH selected in the range of 2 to 3.

A particularly suitable aqueous dispersion of an acrylic emulsion polymers according to the present invention is commercially available under the Tradename TILAMAR^{®} *Fix* A140 (INCI: acrylates copolymer, Chemical Name: polymer with 2-methyl-2-propenoic acid, butyl 2-methyl-2-propenoate, and ethyl 2-propenoate, CAS Number: 26715-43-5) at DSM Nutritional Products Ltd.

The acrylic emulsion polymer can furthermore be used as such or in its neutralised form. Neutralization can be achieved by addition of an appropriate neutralization agent such as an alkali metal hydroxide such as sodium hydroxide, potassium hydroxide or ammonium hydroxide or an amine such as 2-amino-2-methyl-1-propanol, triiso-propanolamine, triethanolamine, tromethamine, 2-amino-2-ethylpropane-1,3-diol or 3-diethylamino-1-propylamine such as particularly with 2-amino-2-methyl-1-propanol which is e.g. available as AMP 95 at DOW.

In a particularly preferred embodiment, the acrylic emulsion polymer is incorporated into the skin cleansing composition according to the present invention without neutralization, i.e. in its acid form.

Particularly suitable skin cleansing formulations according to the invention are liquid soaps, shower gels as well as facial and body cleansers.

The pH of the liquid soaps, shower gels as well as facial and body cleansers is preferably selected in the range of 4.5 to 7, more preferably in the range of 5.0 to 6.5.

The skin cleansing compositions according to the invention preferably comprise from 50 to 90 wt.-%, more preferably from 60 to 80 wt.-% of water, based on the total weight of the skin cleansing compositions.

Examples of suitable anionic surfactants according to the invention are the alkyl sulfates, alkyl ether sulfates, alkylaryl sulphonates, alkanoyl isothionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium or mono-, di- or triethanolamine salts. The alkyl and acyl groups generally contain from 8 to 18, preferably from 10 to 16, carbon atoms and may be unsaturated. The alkyl ether sulfates, alkyl ether sulphosuccinates, alkyl ether phosphates and alkyl ether carboxylic acids and salts thereof may contain from 1 to 20 ethylene oxide or propylene oxide units per molecule.

In particular, the anionic surfactants are selected from sodium oleyl succinate, ammonium lauryl sulphosuccinate, sodium lauryl sulfate, sodium lauryl ether sulfate (also known as sodium laureth sulfate, SLES), sodium lauryl ether sulphosuccinate, ammonium lauryl sulfate (ALS), ammonium lauryl ether sulfate (ammonium laureth sulfate), sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate or mixtures thereof. Preferred anionic surfactants are sodium lauryl sulfate, sodium lauryl ether sulfate (n) EO, (where n is from 1 to 4, in particular n is 3), sodium lauryl ether sulphosuccinate (n) EO, (where n is from 1 to 4, in particular n is 3), ammonium lauryl sulfate, ammonium lauryl ether sulfate (n) EO, (where n is from 1 to 4, in particular n is 3) or mixtures thereof.

In all embodiments of the present invention the anionic surfactant is preferably selected from sodium lauryl sulfate, ammonium lauryl sulfate, sodium lauryl ether sulfate, ammonium lauryl ether sulfate, sodium lauroyl sarconisate, sodium oleylsuccinate, ammonium lauryl sulfosuccinate, sodium dodecylbenzol sulfonate and/or triethanolamine dodecylbenzol sulfonate or mixtures thereof. Most preferably in all embodiments of the invention the anionic surfactant is selected from sodium lauryl sulfate, ammonium lauryl sulfate, sodium lauryl ether sulfate (also known as sodium Laureth sulfate) and/or ammonium lauryl ether sulfate. Particularly preferred in all embodiments of the invention is the use sodium lauryl ether sulfate e.g. available as Texapon^{®} NSO-B at Caelo.

Further suitable surfactants are sodium parethC12-13 sulfate and sodium myreth-sulfate sodium laureth-11 carboxylate and sodium laureth-6 carboxylate.

The total amount of the anionic surfactant(s) in the skin cleansing compositions according to the present invention is preferably selected in the range of 0.1 to 25 wt.-%, more preferably in the range of 2 to 20 wt.-%, most preferably in the range of 3.5 to 15 wt.-% such as in particular in the range of 3.5 to 7 wt.-% based on the total weight of the skin cleansing compositions.

The skin cleansing compositions according to the present invention preferably include at least one co-surfactants.

Examples of co-surfactants are nonionic surfactants, which can be included in an amount ranging from 0.5 to 8 wt.-%, preferably from 2 to 5 wt.- % based on the total weight of the skin cleansing composition. For example, representative nonionic surfactants that can be included into skin cleansing compositions according to the invention include condensation products of aliphatic (C₈ - C₁₈) primary or secondary linear or branched chain alcohols with alkylene oxides, usually ethylene oxide and generally having from 6 to 30 ethylene oxide groups. Other representative nonionic surfactants include mono- or di-alkyl alkanolamides such as e.g. coco mono- or di- ethanolamide and coco mono-isopropanolamide. Further nonionic surfactants which can be included in skin cleansing compositions of the invention are the alkyl polyglycosides (APGs). Typically, the APG is one which comprises an alkyl group connected (optionally via a bridging group) to a block of one or more glycosyl groups such as e.g. Oramix™ NS 1O ex Seppic; PLANTACARE^{®} 818UP, PLANTACARE^{®} 1200 and PLANTACARE^{®} 2000 ex Cognis.

Another example of a co-surfactant is an amphoteric or zwitterionic surfactant, which can be included in an amount ranging from 0.5 to about 8 wt.-%, preferably from 1 to 5 wt.-% most preferably in the range of 1 to 2 wt.-% based on the total weight of the skin cleansing composition. Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in skin cleansing compositions according to the invention include lauryl amine oxide, cocodimethyl sulphopropyl betaine, lauryl betaine, cocamidopropyl betaine (CAPB), sodium cocoamphoacetate and disodium cocoamphodiacetate. Particularly preferred amphoteric or zwitterionic surfactants to be used in the skin cleansing compositions of the present invention are cocamidopropyl betaine, cocoamphoacetate or cocoamphodiacetate such as most preferably sodium cocoamphoacetate.

In a particular advantageous embodiment the skin cleansing composition contains only sodium lauryl ether sulfate and sodium cocoamphoacetate as surfactants.

In a further preferred embodiment the skin cleansing composition comprises at least one additional active ingredient selected from the group consisting of glucose, fructose, saccaride isomerate, glycerin and/ or panthenol, preferably from glucose, fructose, saccaride isomerate, and/ or panthenol, most preferably from saccaride isomerate, and/ or panthenol as this further enhances the soft and smooth skin feel.

Saccaride isomerate is a well-known water-binding agent and emollient used for long-lasting moisturization consisting mainly of glucose and fructose. Saccaride isomerate is e.g. commercially available under the trademark name Pentavitin at DSM Nutritional Products Ltd.

Panthenol (CAS 81-13-0) is e.g. commercially available as D-Panthenol or DL-Panthenol at DSM Nutritional Products Ltd.

The total amount(s) of the additional skin active ingredient is preferably selected in the range of 0.05 to 5 wt.-%, preferably in the range of 0.1 to 3 wt.-%, most preferably in the range of 0.3 to 2 wt.-%.

Thus, in a particular preferred embodiment the present invention is directed to a skin cleansing composition comprising at least one anionic surfactant and and an active ingredient selected from the group consisting of glucose, fructose, saccaride isomerate, glycerin and/ or panthenol and an acrylic emulsion polymer obtainable by emulsion polymerization of a monomer composition consisting of a mixture of methacrylic acid, ethyl acrylate and n-butyl methacrylate, characterized in that the acrylic emulsion polymer is incorporated into the formulation in a concentration ranging from 0.1 to 2 wt.-% (based on solids) based on the total weight of the composition. More preferably, the anionic surfactant consists only of sodium lauryl ether sulfate and sodium cocoamphoacetate and the active ingredient is saccaride isomerate, and/ or panthenol.

The invention is further illustrated with reference to the following, non-limiting examples, in which all percentages are by weight based on total weight unless otherwise specified.

### Example 1

**Table 1: Shower gel**

| Tradename | INCI | Placebo | Sample 1 | Sample 2 | Sample 3 |
|---|---|---|---|---|---|
| | | Wt.-% | | | |
| Water | Aqua | Ad 100 | Ad 100 | Ad 100 | Ad 100 |
| Texapon NSO BZ (28% solution) | Sodium laureth sulfate | 13.5 | 13.5 | 13.5 | 13.5 |
| Miranol Ultra C 32 (40% solution) | Sodium Cocoamphoacetate | 4 | 4 | 4 | 4 |
| Euxyl PE 9101 | Phenoxyethanol | 0.4 | 0.4 | 0.4 | 0.4 |
| NaCl | Sodium chloride | 2 | 2 | 2 | 2 |
| Citric acid anhydrous | Citric acid | 0.4 | 0.4 | 0.4 | 0.4 |
| Antil 171 | PEG-18 Glyceryl Oleate/Cocoate | 2 | 2 | 2 | 2 |
| TILAMAR^{®} FIX A140 (40% solids) | Acrylates copolymer, methylisothiazolinone | | 3 (1.2 based on solids) | 1.5 (0.6 based on solids) | 3 (1.2 based on solids) |
| Pentavitin^{®} | Sacharide isomerate Aqua Citric acid Sodium Citrate | | | 0.75 | 1.5 |
| Panthenol (75%) | panthenol | | | 0.25 | 0.5 |
| Perfum | | 0.5 | 0.5 | 0.5 | 0.5 |

### Sensory evaluation

The shower gels as outlined in Table 1 were tested in a blind study with a sensorial panel consisting of 3 persons under the following conditions:
The evaluation takes part on the inner forearm; the panel leader applies 50 µL of the respective sample on pre-wetted skin.
Evaluator spreads the product within a defined circle of 5 cm diameter using index or middle finger, circular motion, rate of 2 rotations/second for 30 seconds in total. Afterwards the product is rinsed off for 30 seconds under running water. The smoothness is scaled on a scale from + to +++ in comparison to the placebo, which leaves a sticky skin feel. All samples showed no stickiness and an increased smoothness of the skin. Furthermore, as can be retrieved from table 2 sample 2 and 3, the addition of further skin care actives, i.e. pentavitin and panthenol resulted in an even more pronounced smoothness of the skin compared to the base containing no and sample 1 containing only the acrylic emulsion polymer.

**Table 2**

| **Product** | **Sample 1** | **Sample 2** | **Sample 3** |
|---|---|---|---|
| Smoothness of the skin | + | ++ | +++ |

## Claims

1. A skin cleansing composition comprising at least one anionic surfactant and an acrylic emulsion polymer obtainable by emulsion polymerization of a monomer composition consisting of a mixture of methacrylic acid, ethyl acrylate and n-butyl methacrylate, **characterized in that** the acrylic emulsion polymer is incorporated into the composition in a concentration ranging from 0.1 to 2 wt.-% based on the total weight of the composition.

2. The skin cleansing composition according to claim 1, **characterized in that** the amount of the acrylic emulsion polymer is selected in the range of 0.25 to 1.8 wt.-% based on the total weight of the composition.

3. The skin cleansing composition according to claim 1, **characterized in that** the amount of the acrylic emulsion polymer is selected in the range of 0.5 to 1.3 wt.-% based on the total weight of the composition.

4. The skin cleansing composition according to any one of claims 1 to 3, **characterized in that** the acrylic emulsion polymer is obtainable by emulsion polymerisation of a monomer composition consisting essentially of 10-30 wt.-% of methacrylic acid, 5-15 wt.-% of ethyl acrylate and 60-80 wt.-% of n-butyl methacrylate.

5. The skin cleansing composition according to any one of claims 1 to 4, **characterized in that** the acrylic emulsion polymer is obtainable by emulsion polymerisation of a monomer composition consisting essentially of 18-23 wt.-% of methacrylic acid, 9-11 wt.-% of ethyl acrylate and 67-72 wt.-% of n-butyl methacrylate.

6. The skin cleansing composition according to any one of claims 1 to 5, **characterized in that** the acrylic emulsion polymer is incorporated into the composition without neutralization.

7. The skin cleansing composition according to any one of claims 1 to 6, **characterized in that** the anionic surfactant is selected from the group consisting of from sodium lauryl sulfate, ammonium lauryl sulfate, sodium lauryl ether sulfate and/or ammonium lauryl ether sulfate.

8. The skin cleansing composition according to claim 7, **characterized in that** the anionic surfactant is sodium lauryl ether sulfate.

9. The skin cleansing composition according to claim 1 or 8, **characterized in that** the total amount of the anionic surfactant(s) is selected in the range of 0.1 to 25 wt.-%.

10. The skin cleansing composition according to any one of claims 1 to 9, **characterized in that** an additional co-surfactant is present.

11. The skin cleansing composition according to claim 10, **characterized in that** the additional co-surfactant is sodium cocoamphoacetate.

12. The skin cleansing composition according to any one of claims 1 to 11, **characterized in that** the skin cleansing composition is a shower gel.

13. The skin cleansing composition according to any one of claims 1 to 11 **characterized in that** the composition comprises at least one additional active ingredient selected from the group consisting of glucose, fructose, saccaride isomerate, glycerin and/ or panthenol.

14. The skin cleansing composition according to claim 13, **characterized in that** the total amount of the at least one additional active ingredient is selected in the range of 0.05 to 5 wt.-%.

15. Use of an acrylic emulsion polymer obtainable by emulsion polymerization of a monomer composition consisting of a mixture of methacrylic acid, ethyl acrylate and n-butyl methacrylate as skin conditioning agent.
